# EUROPEAN PATENT APPLICATION

(11) **EP 4 000 570 A1**
(43) Date of publication of application: **25.05.2022**
(21) Application number: 21152916.9
(22) Date of filing: 22.01.2021
(51) Int. Cl.: A61F 13/53, A61F 13/533, A61F 13/536

(54) **ABSORBENT CORES COMPRISING FOAM MATERIAL**

(30) Priority: 20.11.2020 EP 20209003
(71) Applicant: Ontex BV, 9255 Buggenhout (BE); Ontex Group NV, 9320 Erembodegem (BE)
(72) Inventor: Staelens, Eva, 9900 Eeklo (BE); Cobbaert, Dries, 1770 Liedekerke (BE); Dhooge, Lieven, 9940 Ertvelde (BE); Koralalage, Chaminda, Hereford, HR1 1FB (GB); Davidson, Roderick, Petersfield, Hants GU32 1JP (GB)
(74) Representative: Larangé, Françoise

(57) **Abstract**

The present invention relates to absorbent cores for use in absorbent articles, preferably of the disposable personal hygiene type. It discloses absorbent cores comprising a foam layer incorporating a nonwoven fabric and having a top surface and a bottom surface, said foam layer top surface comprising one or more indented compressed portions and one or more raised non-compressed portions. The foam layer has openings extending through the foam and the nonwoven fabric. The absorbent core further comprises, joined to the bottom surface of the foam layer, a nonwoven comprising superabsorbent polymers. Absorbent cores of the present invention provide a fast acquisition of body exudates and a good liquid retention capacity. This is further achieved in a simple and cost-effective way without complex and considerable foam formulation and processing requirements.

## Description

### TECHNICAL FIELD

The present invention relates to absorbent cores comprising foam material, absorbent articles comprising said cores and methods of making. Such absorbent articles include dressings, wound dressings, pads, lactation pads, heel pads, sanitary products, hygiene products and maternity towels. In particular, the present invention relates to absorbent articles of the disposable personal hygiene type. Disposable personal hygiene absorbent articles herein are typically selected from diapers (for baby or adult incontinence), pants (for baby or adult incontinence), sanitary napkins, sanitary towels, light incontinence towels and pads, and combinations thereof. Disposable personal hygiene absorbent articles typically include a liquid permeable topsheet, a liquid impermeable backsheet, and an absorbent core enclosed between the topsheet and the backsheet; the present invention particularly relates to such absorbent cores comprising foam material.

### BACKGROUND

The development of highly absorbent articles for use as wound dressings, disposable diapers, adult incontinence pads and briefs, and sanitary napkins, is the subject of substantial commercial interest. The ability of such products to acquire, distribute, and store fluids such as are found in body exudates (e.g., urine, sweat, feces, liquid stools, blood and menses) is obviously important to their function. Historically, this has been primarily achieved by using for example, as acquisition and distribution layers, high lofty nonwovens and, as absorbent core, cellulosic fibers and/or superabsorbent particles (generally lightly crosslinked partially neutralized polyacrylic acid that forms a gel when exposed to free water). This approach has, however, encountered a number of difficulties in achieving efficient removal of fluid from the body of the wearer and storage away from the wearer, leading to visible stains (of e.g. menses, liquid stool or urine) on the body facing side of the articles, and in achieving proper flexibility and comfort, i.e. in achieving to provide a product which moves together with the user's movements and which closely follows the shape of the body when being worn.

In the past decade intensive research and development has been carried out in the development of foams particularly designed for disposable personal hygiene products, for example as described in US5817704, US5856366, US5869171, US6207724, US2002128338, and US2005192365. All such attempts have focused on optimization of a number of foam properties, such as capillarity, density, cell size and the like, by particular selection of foams, chemical composition and processing techniques. And these developments have led to successful commercial products such as Always Infinity@, manufactured by the Procter and Gamble Company, having an absorbent core made of two layers of foam having different pore size. Always Infinity@ products however suffer from high acquisition times, meaning that the speed at which the absorbent article absorbs liquid is low.

WO2017212292 from Sentient Foams Ltd describes absorbent foam products, in particular wound dressings and sanitary products made of aliphatic polyurethane foam and having a profiled surface. The profiled absorbent foam product is said to be comfortable, to allow sufficient air flow to the dressed or covered area, and to exhibit an advantageous capillary motor effect during the absorption of fluid.

We have noted that some products of the prior art, although offering good fluid distribution and absorbency level, suffer from high acquisition times, in addition to bad rewet.

Therefore a need still exists for further specifically optimizing efficient fast absorption of body exudates (which helps in preventing leaks), and reduced rewet (which offers a drier feeling for the wearer), at a reduced cost. Indeed, some of the disadvantages of foam structures of the prior art include complex processes of making as well as high cost.

The present invention is directed at solving the drawbacks still present in the current state of the art.

### SUMMARY OF THE INVENTION

In a first aspect, the present disclosure relates to an absorbent core for an absorbent article according to claim 1. Such absorbent core comprises a foam layer incorporating a nonwoven fabric and having a top surface and a bottom surface. Said foam layer top surface comprises one or more indented compressed portions and one or more raised non-compressed portions. The foam layer has openings extending from said top surface to said bottom surface, through the foam and the nonwoven fabric. The absorbent core further comprises, joined to the bottom surface of the foam layer, a nonwoven comprising superabsorbent polymers.

The product of the present invention overcomes the problems of prior art products, as it provides a fast acquisition of body exudates within the product and a good liquid retention capacity, by providing at the same time a combination of low acquisition time and low rewet. This is further achieved in a simple and cost-effective way without complex and considerable foam formulation and processing requirements.

Other objects and advantages of this invention will become apparent hereinafter.

### DESCRIPTION OF FIGURES

Fig. 1 is a top plan view of an exemplary absorbent core according to the present invention.
Fig. 2 is a top view picture of sanitary napkins, with on the left side a product according to the present invention and on the right side an Always Infinity@ product.
Fig. 3 schematically represents a method of manufacturing absorbent cores according to the present invention.
Fig. 4 schematically represents a method of manufacturing a nonwoven comprising superabsorbent polymers used in the present invention.

### DETAILED DESCRIPTION OF THE INVENTION

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1 % or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein. Herein, "comprise" and "include" mean that other elements and/or other steps which do not affect the end result can be added. Each of these terms encompasses the terms "consisting of" and "consisting essentially of".

The expression "in direct contact" means that the elements referred to are touching each other. Some adhesive may be present between the two elements in contact. This adhesive may be present as a full surface layer or as a zoned layer, where only regions (e.g. stripes, patterns) have adhesive. The presence of adhesive does not change the definitions of direct contact mentioned above; adhesive is simply not considered when assessing if there is direct contact.

"Anisotropy or anisotropic" as used herein means that the element referred to (e.g. the pores or cells of the foam(s)) are elongate in shape (i.e. have a non-uniform, non-homogeneous or non-spherical shape) and comprise one, preferably only one, longest dimension (i.e. a longest length being greater than all other dimensions forming said element). Ideal isotropic elements have an average anisotropy ratio R of 1. Anisotropic elements have an average anisotropy ratio R of greater than 1, as measured according to the method described herein.

The expression "% by weight" (weight percent or %wt), here and throughout the description unless otherwise defined, refers to the relative weight of the respective component based on the overall weight of the formulation.

The recitation of numerical ranges by endpoints includes all numbers and fractions subsumed within that range, as well as the recited endpoints.

The expression "absorbent article" or "personal hygiene articles" or "personal hygiene absorbent articles" refers to articles which absorb and contain body exudates or discharges such as body fluids, and is intended to include for example sanitary napkins, pantiliners, diapers, pants, and incontinence pads (and other articles worn in the crotch region of a garment).

The expression "disposable" refers to articles which are intended to be discarded after a single use, composted, or otherwise disposed of in an environmentally compatible manner. (That is, they are not intended to be laundered or otherwise restored or reused as an absorbent article.)

The expression "sanitary napkin" refers to articles which are worn by females adjacent to the pudendal region which are intended to absorb and contain the various exudates which are discharged from the body (e.g., blood, menses, and urine).

The "absorbent medium" or "absorbent core" or "absorbent body" is the absorbent structure disposed between the topsheet and the backsheet of the absorbent article in at least the crotch region of the absorbent article and is capable of absorbing and retaining liquid body exudates. The size and the absorbent capacity of the absorbent medium should be compatible with the size of the intended wearer and the liquid loading imparted by the intended use of the absorbent article. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers or superabsorbent polymer particles, foam, absorbent nonwoven materials (e.g. nonwoven comprising at least 50 % cotton) or the like, or any combination of two or more of these absorbent materials like "airlaid cores", also called "airlaid paper" made of a thin airlaid layer onto which SAP are adhered. It is common to combine cellulosic fluff pulp with superabsorbent polymers in an absorbent material. The absorbent core may comprise one or more layers of absorbent material stacked on top of each other.

The term "topsheet" refers to a liquid permeable material sheet forming the inner cover of the absorbent article and which in use is placed in direct contact with the skin of the wearer. The topsheet is typically employed to help isolate the wearer's skin from liquids held in the absorbent structure.

The topsheet can comprise a nonwoven material, e.g. spunbond, meltblown, carded, hydroentangled, wetlaid etc. Suitable nonwoven materials can be composed of man-made fibres, such as polyester, polyethylene, polypropylene, viscose, rayon etc. or natural fibers, such as wood pulp or cotton fibres, or from a mixture of natural and man-made fibres. Suitable nonwoven materials can be apertured. The topsheet material may further be composed of two fibres, which may be bonded to each other in a bonding pattern. Further examples of topsheet materials are porous foams, apertured plastic films, laminates of nonwoven materials and apertured plastic films etc. The materials suited as topsheet materials should be soft and non-irritating to the skin and be readily penetrated by body fluid, e.g. urine or menstrual fluid. The inner coversheet may further be different in different parts of the absorbent article. The topsheet fabrics may be composed of a substantially hydrophobic material, and the hydrophobic material may optionally be treated with a surfactant or otherwise processed to impart a desired level of wettability and hydrophilicity.

The term "backsheet" refers to a material forming the outer cover of the absorbent article. The backsheet prevents the exudates contained in the absorbent structure from wetting articles such as bedsheets and undergarments which contact the disposable absorbent article. The backsheet may be a unitary layer of material or may be a composite layer composed of multiple components assembled side-by-side or laminated. The backsheet may be the same or different in different parts of the absorbent article. At least in the area of the absorbent medium the backsheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The backsheet may be partially or fully made of bio-based materials and/or include natural fibers. It may be recyclable, biodegradable and/or compostable. The backsheet material may be breathable so as to allow vapour to escape from the absorbent material, while still preventing liquids from passing there through. Examples of breathable backsheet materials are porous polymeric films, nonwoven laminates of spunbond and meltblown layers and laminates of porous polymeric films and nonwoven materials.

The terms "superabsorbent" or "high-absorbency" refers to materials that are capable of absorbing at least 10 times their own weight in liquid. Superabsorbent materials suitable for use in the present disclosure are known to those skilled in the art, and may be in any operative form, such as particulate form, fibers and mixtures thereof. Generally stated, the "superabsorbent material" can be a water-swellable, organic or inorganic, generally water-insoluble, hydrogel-forming polymeric absorbent material, which is capable of absorbing at least about 15, suitably about 30, and possibly about 60 times or more its weight in physiological saline (e.g. saline with 0.9 wt % NaCI). The superabsorbent material may be natural (i.e. partially or fully made from renewable resources and/or biodegradable), synthetic or from modified natural polymers and materials. The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal and ammonium salts of polyacrylic acid and polymethacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, maleic anhydride copolymers with vinyl ethers and alpha-olefins, poly(vinyl pyrrolidone), poly(vinylmorpholinone), poly(vinyl alcohol), and the like, and mixtures and copolymers thereof. Other suitable hydrogel-forming polymers include hydrolyzed acrylonitrile grafted starch, acrylic acid grafted starch, and isobutylene maleic anhydride copolymers and mixtures thereof, methyl cellulose, carboxymethyl cellulose, hydroxypropyl cellulose, and the natural gums, such as alginates, xanthan gum, locust bean gum and the like. The hydrogel-forming polymers may be inorganic materials, such as silica gels, or organic compounds such as cross-linked polymers. The term "cross-linked" refers to any means for effectively rendering normally water-soluble materials substantially water insoluble but swellable. Such means can include, for example, physical entanglement, irradiation, crystalline domains, covalent bonds, ionic complexes and associations, hydrophilic associations, such as hydrogen bonding, and hydrophobic associations or Van der Waals forces. Mixtures of natural and wholly or partially synthetic absorbent polymers can also be used. Synthetic hydrogel-forming materials typically are xerogels which form hydrogels when wetted. The term "hydrogel", however, has commonly been used to also refer to both the wetted and unwetted forms of the material. The superabsorbent material may be in any of a wide variety of geometric forms. As a general rule, it is preferred that the superabsorbent material be in the form of discrete particles. However, the superabsorbent material may also be in the form of fibres, flakes, rods, spheres, needles, spiral or semi-spiral, cubic, rod-like, polyhedral, or the like. Conglomerates of particles of superabsorbent material may also be used. The superabsorbent material may suitably be included in an appointed storage or retention portion of the absorbent system, and may optionally be employed in other components or portions of the absorbent article. The superabsorbent material may be included in the absorbent layer or other fluid storage layer of the absorbent article in an amount of from about 5 to about 100 weight percent and desirably from about 30 to about 100 weight percent based on the total weight of the absorbent core. The distribution of the high-absorbency material within the different portions of the absorbent core can vary depending upon the intended end use of the absorbent core. The high-absorbency material may be arranged in a generally discrete layer within the matrix of hydrophilic fibres. Alternatively, the absorbent core may comprise a laminate of fibrous webs and high-absorbency material or other suitable means of maintaining a high-absorbency material in a localized area.

"Superabsorbent polymers" or "SAP" refer to water-swellable, water-insoluble organic or inorganic materials capable, under the most favorable conditions, of absorbing at least about 10 times their weight, or at least about 15 times their weight, or at least about 25 times their weight in an aqueous solution containing 0.9 weight percent sodium chloride. In absorbent articles, such as diapers, incontinent diapers, etc., the particle size is typically ranging between 100 to 800 µm, preferably between 300 to 600 µm, more preferably between 400 to 500 µm.

By the terms "particle", "particles", "particulate", "particulates" and the like, it is meant that the material is generally in the form of discrete units. The units can comprise granules, powders, spheres, pulverized materials or the like, as well as combinations thereof. The particles can have any desired shape such as, for example, cubic, rod-like, polyhedral, spherical or semi-spherical, rounded or semi-rounded, angular, irregular, etc. Shapes having a large greatest dimension/smallest dimension ratio, like needles, flakes and fibers, are also contemplated for inclusion herein. The terms "particle" or "particulate" may also include an agglomeration comprising more than one individual particle, particulate or the like. Additionally, a particle, particulate or any desired agglomeration thereof may be composed of more than one type of material.

The term "polymer" generally includes, but is not limited to, homopolymers, copolymers, such as, for example, block, graft, random and alternating copolymers, terpolymers, etc. and blends and modifications thereof. Furthermore, unless otherwise specifically limited, the term "polymer" shall include all possible geometrical configurations of the material. These configurations include, but are not limited to, isotactic, syndiotactic and random symmetries.

The term "nonwoven fabric or web or layer" means a sheet material having a structure of individual fibers or threads which are interlaid, but not in a regular manner such as occurs with knitting or weaving processes. Nonwoven fabrics or webs or layers have been formed from many processes such as for example, meltblowing processes, spunbonding processes, and bonded carded web processes.

The expression "body surface" refers to surfaces of absorbent articles and/or their component members which face the body of the wearer, while the term "garment surface" refers to the opposite surfaces of the absorbent articles and/or their component members that face away from the wearer when the absorbent articles are worn. Absorbent articles and components thereof, including the topsheet, backsheet, absorbent core, and any individual layers of their components, have a body surface and a garment surface.

The expression "substantially parallel" as used herein means that the element referred to is within 30°, preferably within 15°, more preferably within 10°, most preferably within 5°, from the axis, plane or element referred to.

The expression "substantially perpendicular" as used herein means that the element referred to is within 30°, preferably within 15°, more preferably within 10°, most preferably within 5°, from the axis, plane or element referred to.

Embodiments of the articles and processes according to the disclosure will now be described. It is understood that technical features described in one or more embodiments maybe combined with one or more other embodiments without departing from the intention of the disclosure and without generalization therefrom.

According to the invention, the absorbent core comprises a foam layer incorporating a nonwoven fabric and having a top surface and a bottom surface. Said foam layer top surface comprises one or more indented compressed portions and one or more raised non-compressed portions. The foam layer has openings extending from said top surface to said bottom surface, through the foam and the nonwoven fabric. The absorbent core further comprises, joined to the bottom surface of the foam layer, a nonwoven comprising superabsorbent polymers.

By specifying that a nonwoven fabric is incorporated within the foam layer, we mean herein that the nonwoven fabric is included within the foam, i.e. surrounded by foam on both its main surfaces, meaning that the upper main surface of the nonwoven is in direct contact with foam and the lower main surface of the nonwoven is in direct contact with foam. The nonwoven fabric incorporated within the foam may ensure foam stability when the foam starts to swell due to liquid absorption. In particular the nonwoven fabric may prevent expansion of the foam, upon absorption of fluid, in the plane of the nonwoven fabric. The nonwoven fabric may also favour distribution of the liquid, to transport the body fluid away from the initial impingement zone and use as much as possible the whole surface of the absorbent zone of the article. The width and/or length of the nonwoven fabric may be identical or similar or different than the width and/or length of the foam layer. In some advantageous embodiments, the nonwoven fabric has an area similar to the area of the foam layer.

The foam layer top surface comprises one or more indented compressed portions and one or more raised non-compressed portions. Such profiled surface creates a so-called "hill-and-valley" effect on the foam layer top surface which may help the fluid distribution in all directions of the main plane of the core. By "non-compressed" we mean herein that the raised portions are substantially non-compressed, or not voluntary compressed, or preferably, significantly less compressed than the compressed portions.

In an embodiment the foam layer bottom surface also comprises one or more indented compressed portions and one or more raised non-compressed portions. The resulting profiled surface on the foam layer bottom surface may be identical or similar or different than the profiled surface on the foam layer top surface. Preferably, the resulting profiled surface on the foam layer bottom surface is similar to the profiled surface on the foam layer top surface and both are in face to face relationship, meaning that an indented compressed portion on the top surface faces an indented compressed portion on the bottom surface, and a raised non-compressed portion on the foam layer top surface faces a raised non-compressed portion on the foam layer bottom surface. The presence of a profiled surface on the foam layer bottom surface may help improving the visual appearance of the article in use, reducing exudates stains visibility and/or creating minimal "blood-spots" on the top surface of the core.

According to the invention, the foam layer has openings extending from said top surface to said bottom surface, through the foam and the nonwoven fabric, i.e. the foam layer openings extend across the entire thickness of the foam layer. These openings allow the exudates to directly and quickly flow from the top surface of the foam layer along the thickness direction towards the bottom surface of the foam layer and the further nonwoven layer joined to the bottom surface of the foam layer. We have found that such openings greatly participate in the improved acquisition time of the present invention. For even better results, the openings are preferably present in the indented compressed portions. The openings may further extend across the entire thickness of the nonwoven comprising superabsorbent polymers, thereby extending across the entire thickness of the absorbent core. This may be easier for manufacturing purposes, whilst still providing the same advantages in terms of acquisition time.

According to the invention, in addition to the foam layer, the absorbent core further comprises, joined to the bottom surface of the foam layer, a nonwoven comprising superabsorbent polymers. We have found that the presence of superabsorbent polymers is essential to provide the degree of absorption required for some articles of the invention, for example an ISO-absorption according to ISO 11948-1:1996 higher than 65 g in an embodiment of a sanitary napkin. However we have found that having superabsorbent polymers integrated within the foam creates various problems. Firstly, a problem of curling, the absorbent article tending to become curled while absorbing liquid. Secondly, we have noted that some superabsorbent polymers could get out of the foam and out of the absorbent article during use, which is totally unacceptable for the wearer. Thirdly, without wishing to be bound by theory, we believe that superabsorbent polymers, whilst entrapped in a pore of a foam, may not act properly and not play their role correctly, providing reduced properties. We have found that associating a nonwoven comprising superabsorbent polymers with the foam, as part of the core, may instead provide an increased absorption capacity without any adverse effect of deformation. This additional nonwoven being in fluid communication with the foam layer above, it can offer an additional reservoir for the absorption of liquids.

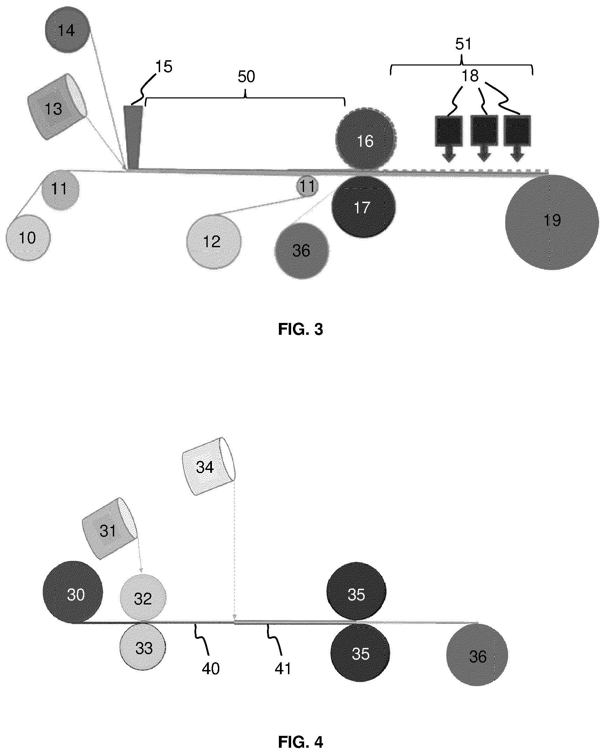

Such nonwoven comprising superabsorbent polymers may be selected for example from a nonwoven comprising superabsorbent fibres, a SAP paper readily available on the market, a nonwoven having clusters of superabsorbent polymer particles on a surface of the nonwoven and an adhesive for covering and immobilizing the superabsorbent polymer particles (as described in WO2008/155699 A1 figure 3 for example), or more generally a nonwoven having attached on one of its main surfaces superabsorbent polymer particles. Preferably the superabsorbent polymer particles are facing the foam layer and the nonwoven constitutes an external face of the absorbent core. In an embodiment of a sanitary napkin, the quantity of superabsorbent polymer particles may advantageously be between 20 and 50 gsm, preferably between 25 and 45 gsm.

Advantageously the superabsorbent polymers may be present in a pattern wherein the superabsorbent particles do not face the openings in the foam layer or are not immediately adjacent to any opening. This may reduce the risk of any leakage of superabsorbent particles through the openings in the direction of the wearer's body, in particular when the particles start swelling during use.

In some embodiments of the invention, a limited amount of superabsorbent polymers, e.g. fibers or particles, may be present in the foam layer, preferably in a portion of the foam layer which is farther away from the foam layer top surface, most preferably in the part of the foam layer which is between the nonwoven fabric and the foam layer bottom surface. The quantity of superabsorbent polymers is preferably sufficiently low to avoid any curling of the absorbent article, for example less than 20 gsm. Advantageously the SAP included in the foam are selected to be permeable for not affecting the acquisition time of the absorbent article, and/or to have a low CRC to avoid a too strong swelling and to reduce the gel blocking effect. The SAP comprised in the nonwoven may on the other hand be selected to have a higher CRC. The SAP included in the foam may thus show a higher permeability and/or a lower CRC than the SAP comprised in the nonwoven.

In an embodiment, the foam comprises, preferably consists of, an open cell polymer foam, more preferably foams selected from the group consisting of polyurethane (PU), poly vinyl alcohol (PVA), polyolefins such as low-density polyethylene (LDPE), ethylene-vinyl acetate (EVA), ethylene butyl acrylate (EBA), open cell silicone foams, natural and synthetic rubbers, and mixtures thereof, most preferred being aliphatic polyurethane foam. These may be either hydrophilic or hydrophobic, at various degrees of hydrophilicity.

In a preferred embodiment, the foam is a polyurethane foam having a density of about 100 to 180 kg/m³. The raised non-compressed portions of the profiled foam may have a density of about 100 to about 140 kg/m³. The indented compressed portions of the profiled foam may have a density of about 140 to about 180 kg/m³.

In a preferred embodiment, the open cells of the foam have an average anisotropy ratio R greater than 1.0, or greater than 1.1, preferably from 1.1 to 3.5, more preferably from 1.2 to 3.0, as measured according to the method described herein. Preferably the anisotropy ratio R in the ranges described above, is for cells having the longest dimension (or length) extending in a direction substantially parallel to the direction that a fluid is expected to travel through a foam layer comprising said cells An advantage of such arrangement is that directionality of capillarity is enabled along the longest length of the anisotropic cells. The anisotropic ratio of each cell is given by dividing the longest cell length by the shortest cell width typically being substantially perpendicular thereto. The average anisotropy ratio R is then given by the sum of said ratios divided by the number of cells n measured. Further details on the method used for calculating the average anisotropy ratio is provided in WO2019053110 A1.

The foam layer may have a maximum thickness of 5, 4, 3 or 2 mm or less. In a preferred embodiment, the thickness of the raised non-compressed portions is between 1.5 and 4 mm, preferably between 2 and 3 mm, and the thickness of the indented compressed portions is between 0.5 and 2 mm, preferably between 0.7 and 1.5 mm.

The profiled surface of the foam layer according to the present invention may be in any compressed, relief pattern or shaped design. However, certain shapes and designs are described below as being associated with particular technical advantages.

The profiled surface may comprise a pattern of connected or interconnected indentations (compressed portions) formed into said surface to provide one or more, such as a plurality, of raised portions (non-compressed portions or hills). The raised (non-compressed) portions may be discrete raised portions. A pattern of connected or interconnected indentations may comprise a first indentation formed into said surface in a first direction and a second indentation formed into said surface in a second direction that is different from the first direction. Optionally a third or even more indentations may be formed into said surface in a third or other directions that are different from the first direction and the second direction. The various indentations may meet or intersect to create the interconnection. The indentations may be in the form of a pattern of connected indentations being non-linear, for example non-linear across their entire length. The pattern of connected indentations may cover more than 10%, 20%, 30%, 40% or 50% of the foam layer surface.

Advantageously, the profiled surface may comprise a central elongate indented compressed portion extending substantially from one edge of the absorbent core to the opposite edge. Such elongated compressed portion may help improving the fluid distribution over the entire surface of the core, in particular in zones further away from the liquid inlet point. Optionally a plurality of additional elongate indented compressed portions may be present, extending from the central indented compressed portion, preferably substantially to one or more other edges of the product. The additional elongate indented compressed portions may be parallel to one another. Each of the additional indented compressed portions may form an acute angle with the central indented compressed portion at the point of its extension from the central indented compressed portion. Accordingly, the profiled surface may comprise V-shaped or herringbone profiling. The herringbone or V-shaped design advantageously provides an in situ drainage system for fluid and/or exudate. The profiled surface may also comprise at least one, advantageously two, generally circular or elliptic indented compressed portion extending substantially around the central elongate indented compressed portion and optional additional elongate indented compressed portions.

The indented compressed portions may have a width of greater than 1 mm, preferably greater than 2 mm. An advantage of this configuration is that liquids can more effectively and quickly be distributed over the foam layer and also to account for deformation of the foam upon swelling in wet conditions so as to retain its general overall shape and limit excess deformation that could lead to discomfort when worn by a subject.

The openings may be of any shape, e.g. circular, ellipsoidal, rectangular, slit, slot, preferably circular. They are preferably present in the indented compressed portions to compensate for the reduced capability of penetration of liquid in these portions. Their maximal dimension, e.g. their diameter in case of circular openings, is advantageously equal or smaller that the width of the indented compressed portions. The openings are preferably present in the central portion of the absorbent core, close to the exudate inlet point, for a rapid intake of fluids towards the bottom surface of the foam layer and the further nonwoven layer joined to the bottom surface of the foam layer. There may be more than 3, 5, 8 or 10 and/or less than 30, 25, 20 or 18 openings.

Absorbent articles according to the present invention may be selected from dressings, wound dressings, pads, lactation pads, heel pads, sanitary products, hygiene products and maternity towels. In particular, they may be absorbent articles of the disposable personal hygiene type. Disposable personal hygiene absorbent articles herein may be selected from diapers (for baby or adult incontinence), pants (for baby or adult incontinence), sanitary napkins, sanitary towels, light incontinence towels and pads, and combinations thereof. In such disposable personal hygiene absorbent articles, the absorbent core of the present invention is typically positioned between a liquid permeable topsheet and a liquid impermeable backsheet, the topsheet facing the top surface of the foam layer and the backsheet facing the nonwoven comprising superabsorbent polymers.

Topsheet and backsheet are generally held together around their peripheries by conventional sealing means like adhesives, embossing, fusing, mechanical sealing and other methods known in the art. This peripheral sealing means may in some instances also include the periphery of the absorbent core. In addition to the peripheral sealing means it is quite common to also bond the layers together at their faces, with for example adhesives. In this context, we have however found that it may be advantageous to not bond the topsheet to the absorbent core, except maybe in the periphery of the absorbent core. This may provide a better run-off efficiency. Such embodiment has however the disadvantage that the foam is less noticeable to the consumer looking at the absorbent article before use, so that the message of innovation, technicality and comfort of the absorbent core comprising foam is less immediately conveyed to the user. We have therefore found that a good compromise could be, in some embodiments, that the topsheet is bonded to the absorbent core in regions of the indented compressed portions, and preferably not elsewhere, except maybe in the periphery of the absorbent core. This still give good results in terms of run-off whilst allowing the user to see the particularities of the absorbent core comprising foam before use.

Suitable topsheets may be manufactured from a wide range of materials including, but not limited to nonwoven materials, apertured formed thermoplastic films, apertured plastic films, and hydroformed thermoplastic films; reticulated thermoplastic films; thermoplastic scrims; and film/nonwoven laminates. Suitable nonwoven materials can be comprised of natural fibers (e.g., wood or cotton fibers), synthetic fibers (e.g., polymeric fibers, such as polyester, polypropylene fibers, and polyethylene, or polyvinylalcohol, starch base resins, nylon, and rayon fibers) or from a combination of natural and synthetic fibers. Apertured formed films and film/nonwoven laminates are generally preferred for the topsheet because they are pervious to liquids and, if properly apertured, have a reduced tendency to allow liquids to pass back through and rewet the wearer's skin. Films may also show better results in terms of run-off. Polyethylene (PE) films and laminates comprising a nonwoven and a PE film are most preferred.

In a second aspect, the present invention provides a method of making an absorbent core as described hereinabove, comprising the steps of:
a) providing a polymer mixture comprising one or more reactants and/or polymers, preferably polyurethane;
b) applying or generating in-situ one or more fluid substances (typically a gas or an aqueous solution) such to promote a cell growth;
c) providing a nonwoven fabric;
d) passing at least some of the polymer mixture through the nonwoven fabric;
e) curing the polymer mixture;
f) profiling the polymer mixture;
g) drying the cured polymer mixture after profiling;
h) providing a nonwoven comprising superabsorbent polymers;
i) joining the nonwoven comprising superabsorbent polymers to the polymer mixture; and
j) creating openings at least across the polymer mixture.

These steps are not limited to the order shown above. In an embodiment, step (b) occurs prior to step (e), which is followed by step (g), which generally occurs last. In an embodiment, step (d) occurs during step (e). In an embodiment, step (d) occurs shortly after the end of step (b). In an embodiment, step (d) occurs early during the start of step (e). In an embodiment, step (d) occurs at any time after the end of step (b), but before the end of step (e). The profiling step (f) takes place before the foam has been dried, i.e. step (g). In an embodiment, step (d) occurs before step (f). Step (i) may advantageously been carried out simultaneously with step (f). Step (j) may preferably be carried out at any moment between the end of phase (e) until the very end of all the steps of the method, including after the drying step (g).

The method comprises a step (d) in which at least some of the polymer mixture is passed through a nonwoven fabric. In an embodiment, step (d) takes place during the curing process of step (e). In an embodiment, at least some of the polymer mixture is extruded through the nonwoven fabric. In an embodiment, only part of the polymer mixture is passed through the nonwoven fabric, and part of the polymer mixture is not passed through the nonwoven fabric.

In the context of the present invention, the term "curing" refers to the stage in the foam production process in which the polymer composition is allowed to cream, gel and rise to its final size. Accordingly, the curing stage ends when the foam has risen to its final size. For example, curing may be considered to have ended prior, such as immediately prior to the (active) drying step (e.g. when the foam enters a hot air tunnel or oven) of step (g) according to the method of the present invention. During the curing step (e), any reactant present in the polymer mixture will begin to produce bubbles of gas, typically carbon dioxide, which will form cavities in the polymer mixture. The curing step (e) may be carried out on a curing means, for example a curing track or curing conveyor. In some embodiments, the whole of the curing step may take between about 30 seconds and about 5 minutes, for example between about 1 and about 3 minutes, preferably about 2 minutes. Preferably, the maximum height of the foam of the present invention at the end of curing is between about 1.5 to about 4 mm. Curing may take place at a temperature of between about 15°C to about 45°C. In preferred embodiments, curing takes place at room temperature (e.g. about 15°C to about 22°C), or at between about 40°C to about 45°C.

The term "drying" in the context of the present invention is meant to refer to the stage of active drying of the foam, i.e. the subjecting of the foam to a specific drying step or drying means intended to remove water or moisture from the foam. This is most commonly effected using heat. Drying may be effected using a drying means, such as a heat tunnel, hot air tunnel, drying oven or source of infrared radiation. In some embodiments, the whole of the drying process may take between about 30 seconds and about 2 minutes, for example between about 45 seconds and about 1 minute 30 seconds, preferably about 1 minute. Drying may take place at a temperature of between about 80°C to about 120°C. In preferred embodiments, drying takes place between about 90°C to about 110°C, preferably at about 100°C. In the context of the present invention, no substantial or quantifiable drying is considered to occur during the curing process. In one embodiment, there may be stepwise changes (e.g. increases) in drying temperature during the drying stage, for example there may be a period of drying at 80°C, followed by a period at 100°C, followed by a period at 120°C.

The profiling step (f) takes place before the foam has been dried, i.e. step (g), preferably immediately before the drying step (g). Profiling may be achieved by any suitable profiling means, for example a roller or plate. The roller or plate may be made of any suitable material. Such a plate or roller may comprise on its surface a template relief pattern to be embossed or imprinted on the foam; when in use said plate or roller comes into contact with the foam. Preferably, the profiling step (f) is carried out using a roller, such as a profiling roller or emboss roller. In other embodiments, the roller may be described by the shape which it embosses or imparts onto the foam. In one embodiment, the roller is heated, for example for a period of 0.5 seconds - 1 second, for example to any temperature above room temperature. The heating may be effected by means of a heating cartridge, such as a cartridge inserted into the roller. In preferred embodiments, all profiling takes place before the drying step. In other embodiments, profiling may also take place after the drying step, or before and after the drying step. In some embodiments, the profiling step (f) takes place after the curing step (e), such as immediately after the curing step. In the same or different embodiment, the profiling step occurs after, such as immediately after, the foam has risen to its final size. In this regard, profiling after the curing step or rise time may be advantageous because the foam may not further distort post-profiling. Preferably, the profiling step takes place after (such as immediately after) the curing step and/or foaming step and/or after the foam has risen to its final size or maximum height, and before (such as immediately before) the drying step. In other words, the profiling step may take place (exactly) between the curing step and the drying step. Accordingly, the profiling step may take place after the entire curing step has taken place, and before any drying has taken place, such as (immediately) before the drying step. In an embodiment, the profiling step takes place after the foam has fully cured and before the drying step. In a related embodiment, the profiling step takes place after the rise time.

In an alternative method, the profiling may be achieved by placing an insert on the curing means, for example a curing track or curing conveyor, which hinders the polymer mixture from occupying this space.

In the embodiment described above where a topsheet is bonded to the absorbent core in regions of the indented compressed portions, the topsheet may be provided just before the profiling step (f) and joined to the polymer mixture during the profiling step.

Step (i) of may advantageously been carried out simultaneously with step (f), the rollers helping with the joining of the nonwoven comprising superabsorbent polymers to the polymer mixture. The subsequent drying step (g) may further help to join the nonwoven comprising superabsorbent polymers to the polymer mixture. Alternatively, the nonwoven comprising superabsorbent polymers may be joined to the polymer mixture after the profiling step, at any moment during the drying step, or after the drying step. The nonwoven comprising superabsorbent polymers may in a further alternative embodiment be joined to the polymer mixture during the curing step, preferably just before the profiling step. In still a further alternative embodiment, the nonwoven comprising superabsorbent polymers can serve as support on which the polymer mixture will cure during the curing step.

Step (j) of creating openings at least across the polymer mixture may be carried out at any moment between the end of phase (e) until the very end of all the steps of the method, including after the drying step (g). In some embodiments, the openings creation step takes place after the curing step (e), such as immediately after the curing step. In the same or different embodiment, the openings creation step occurs after, such as immediately after, the foam has risen to its final size. In this regard, creating openings after the curing step or rise time may be advantageous because the foam may not further distort post-profiling. Preferably, the openings creation step takes place after (such as immediately after) the curing step and/or foaming step and/or after the foam has risen to its final size or maximum height. The selection of the most appropriate moment for the openings creation step may depend if the openings extend across the foam layer only, but not the nonwoven comprising superabsorbent polymers, or if the openings extend across the entire thickness of the absorbent core. It may for example be easier to create the openings in the polymer mixture before it gets joined to the nonwoven comprising superabsorbent polymers in case the openings extend across the foam layer only. A very simple and cost-effective way of creating the openings may be to create these at the very end of the process of making the absorbent core, as the last step of such process.

Step (j) of creating openings may be achieved by any suitable perforating means, for example a roller or plate including pins or needles, or a punch. In an embodiment, the openings are created simultaneously with the profiling step, the profiling roller or emboss roller, having additional pins to create the openings.

Step (j) of creating openings may be carried out after a topsheet has been joined to the absorbent core, so that the openings are created simultaneously in the topsheet and the core, thereby creating an apertured topsheet.

### EXAMPLES

**Example 1** is a sanitary towel (size 1) comprising in sequence:
- a laminated topsheet, 18 gsm, comprising a nonwoven top layer and a perforated PE film as bottom layer, commercially available from Pantex (product reference: PN18N3BI);
- an absorbent core comprising in sequence:
   ∘ a polyurethane foam upper layer, of the type Baymedix^{®} FP505 from Covestro
   ∘ a nonwoven fabric, 20 gsm, commercially available from TWE (product reference: ParaTherm Loft 286/20);
   ∘ a foam lower layer identical to the foam upper layer;
   ∘ a nonwoven, 20 gsm, commercially available from TWE (product reference: ParaTherm Loft 286/20) having adhered on its upper surface 0.30 g of superabsorbent polymers of the type SA60S, commercially available from Sumitomo; and
- a polyethylene non-breathable backsheet of about 22 gsm.

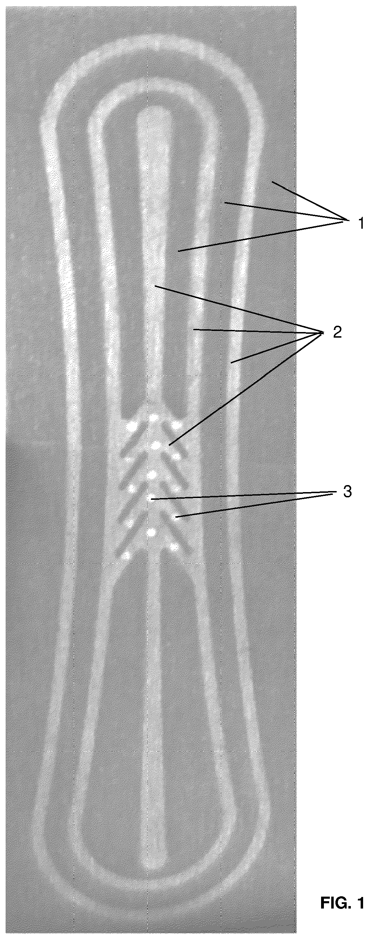

The foam layer top surface comprises indented compressed portions (2) and raised non-compressed portions (1) according to a pattern visible in Figure 1. The absorbent core has openings (3) of about 3 mm diameter, extending across the entire core according to a pattern visible in Figure 1. The topsheet is full surface glued to the core. The sanitary towel thickness measured in a non-compressed region is about 2.2 mm.

Example 1 shows (see Table 1) very good rewet values, in the preferred values of less than 1.5 g for standing rewet and less than 3.5 g for sitting rewet; an excellent acquisition time (AQT) value, in the preferred range of less than 135 seconds; and a limited run-off efficiency, but similar to successful commercial products such as Always Infinity@, manufactured by the Procter and Gamble Company (see comparative example 1).

**Comparative Example 1** is a commercially available product from the Procter and Gamble Company, sold under the trademark Always Infinity@, for regular flow, with flexi-wings, size 1, pack of 18 pads, with code 9075478600 62 075 09:54 MFG 2019-03-16, bought on the US market and tested on 29 May 2019.

Comparative Example 1 shows (see Table 1) very good rewet values but very bad acquisition time, and limited run-off efficiency.

**Example 2** is identical to Example 1, except that the topsheet is a thermocarded nonwoven, 18 gsm, commercially available from Pantex (product reference TB18G1BY).

**Example 3** is identical to Example 2, except that the topsheet is not glued to the core.

The comparison of run-off efficiency values (see Table 1) of Examples 1, 2 and 3, show the advantage of not bonding the topsheet to the absorbent core or using a film/nonwoven laminate as topsheet.

**Comparative Example 2,** not in accordance with the present invention, is identical to Example 2, except that the foam layer top surface is flat and does not comprise indented compressed portions and raised non-compressed portions.

Comparative Example 2 shows (see Table 1) very bad acquisition time, evidencing the advantage of the profiled foam top surface according to the present invention.

| **TABLE 1** | Example 1 | Comp. Ex. 1 | Example 2 | Example 3 | Comp. Ex. 2 |
|---|---|---|---|---|---|
| Standing rewet [g] | 1.16 | 0.86 | - | - | - |
| Sitting rewet [g] | 2.69 | 3.41 | - | - | - |
| AQT[s] | 86 | 444 | 82 | 89 | 468 |
| Run-off efficiency [%] | 58 | 53.7 | 39.9 | 91.9 | - |

**Example 4** is identical to Example 1, except that the topsheet is a perforated nonwoven, 24 gsm, commercially available from Texol (product reference N03L24049).

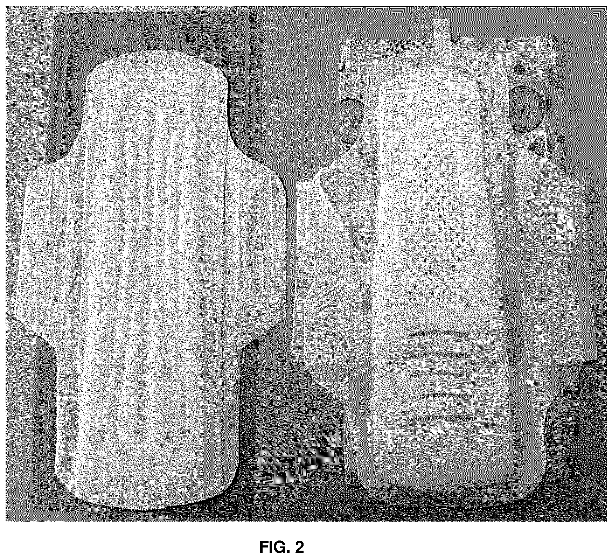

A panel test has been performed in a group of between 5 and 25 panelists, to compare products according to Example 4 of the present invention (left side of Figure 2) and Always Infinity@ products according to Comparative Example 1 (right side of Figure 2). They were asked how was the dry feeling during use. Results are given in Table 2 below. 100% of the panelists judged the dry feeling of Example 4 good or very good, whist only 75% of panelists judged the dry feeling of Comparative Example 1 good or very good. The results show that the present invention advantageously provides a better dry feeling during use.

| **TABLE 2** | Example 4 | Comparative Example 1 |
|---|---|---|
| Very good | 75% | 50% |
| Good | 25% | 25% |
| Moderate | - | 12,5% |
| Bad | - | 12,5% |
| Very bad | - | - |

### Exemplary method

Absorbent cores according to the examples 1-4 have been manufactured following the method schematically represented in Figure 3.

Said method comprised, in order, the steps of:
i. providing a polyurethane mixture (13) and a water phase including sodium bicarbonate and a surfactant to achieve a cell growth (not represented);
ii. providing a nonwoven fabric (14);
iii. pouring the polyurethane mixture (13) and depositing the nonwoven fabric (14) on a casting liner (10) conveyed by rolls (11) and later rewound (12);
iv. passing under a metering bar (15);
v. passing at least some of the polyurethane mixture through the nonwoven fabric (14) whilst curing the polyurethane mixture (the curing step is generally represented as 50) for 2 minutes at 21°C;
vi. providing a nonwoven comprising superabsorbent polymers (36);
vii. joining the nonwoven comprising superabsorbent polymers (36) to the polyurethane mixture and simultaneously profiling the polyurethane mixture between an emboss roller (16) and a plain roller (17);
viii. drying the cured polyurethane mixture (the drying step is generally represented as 51) in an oven (18) for 2 minutes at 90°C; and
ix. winding up (19) the resulting foam layer and nonwoven comprising superabsorbent polymers.

The openings across the absorbent core were created later with a punch, after having cut the resulting foam layer and nonwoven comprising superabsorbent polymers to size for using it as absorbent core for the sanitary towel of the examples.

The nonwoven comprising superabsorbent polymers used in the examples 1-4 has been manufactured following the method schematically represented in Figure 4.

Said method comprised, in order, the steps of:
i. providing a nonwoven (30);
ii. unwinding and passing it through rollers (32,33);
iii. applying glue (31) to one of the rollers (32), so that the glue is applied to the nonwoven and provides a nonwoven having glue on its upper surface (40);
iv. applying superabsorbent polymer particles on the upper surface of the nonwoven, to provide a nonwoven having glue and superabsorbent polymer particles on its upper surface (41);
v. securing the superabsorbent polymer particles to the nonwoven and glue by passing the nonwoven through rollers (35); and
vi. winding up (36) the resulting nonwoven comprising superabsorbent polymers.

### Acquisition time, rewet and run-off efficiency measurements

Acquisition time, rewet and run-off efficiency measurements were carried out according to the Analysis Operating Protocols developed by and available at SGS Courtray Laboratories (Oignies, France). Acquisition time measurements were performed according to Document n° POA/DF4 (revision 02) of 21.09.2011. Rewet measurements were performed according to Document n° POA/DF7-DF8 (revision 05) of 01.02.2012. Run-off efficiency measurements were performed according to Document n° POA/DF9 (revision 01) of 26.06.1998, updated on 30.04.2009.

It is supposed that the present invention is not restricted to any form of realization described previously and that some modifications can be added to the presented example of fabrication without reappraisal of the appended claims.

## Claims

1. An absorbent core for an absorbent article, the absorbent core comprising a foam layer incorporating a nonwoven fabric and having a top surface and a bottom surface, said foam layer top surface comprising one or more indented compressed portions and one or more raised non-compressed portions, **characterised in that** the foam layer has openings extending from said top surface to said bottom surface, through the foam and the nonwoven fabric, and **in that** the absorbent core further comprises, joined to the bottom surface of the foam layer, a nonwoven comprising superabsorbent polymers.

2. An absorbent core according to claim 1, **characterised in that** the foam layer bottom surface comprises one or more indented compressed portions and one or more raised non-compressed portions.

3. An absorbent core according to claim 2, **characterised in that** the compressed and non-compressed portions of the foam layer top and bottom surfaces are similar and in face to face relationship.

4. An absorbent core according to any of the preceding claims, **characterised in that** the openings are present in the indented compressed portions.

5. An absorbent core according to any of the preceding claims, **characterised in that** the openings extend across the entire thickness of the absorbent core.

6. An absorbent core according to any of the preceding claims, **characterised in that** the nonwoven comprising superabsorbent polymers is a nonwoven having attached on one of its main surfaces superabsorbent particles, said particles facing the foam layer.

7. An absorbent core according to claim 6, **characterised in that** the superabsorbent particles are present in a pattern wherein the superabsorbent particles do not face the openings in the foam layer or are not immediately adjacent to any opening.

8. An absorbent core according to any of the preceding claims, **characterised in that** the foam layer comprises superabsorbent polymers in a part of the foam layer which is between the nonwoven fabric and the foam layer bottom surface.

9. An absorbent core according to claim 8, **characterised in that** the superabsorbent polymers in the foam layer have a higher permeability and/or a lower CRC than the superabsorbent polymers comprised in the nonwoven.

10. An absorbent core according to any of the preceding claims, **characterised in that** the foam layer comprises a polymer foam selected from the group consisting of polyurethane; poly vinyl alcohol (PVA); polyolefins selected from the group consisting of low-density polyethylene (LDPE), ethylene-vinyl acetate (EVA), ethylene butyl acrylate (EBA), and mixtures thereof; open cell silicone foams; natural and synthetic rubbers; and mixtures thereof.

11. An absorbent article comprising an absorbent core according to any of the preceding claims, **characterised in that** said absorbent article is selected from the group consisting of dressings, wound dressings, pads, lactation pads, heel pads, sanitary products, hygiene products, disposable personal hygiene absorbent articles and maternity towels.

12. An absorbent article according to claim 11, **characterised in that** the article is a disposable personal hygiene absorbent article and **in that** the absorbent core is positioned between a liquid permeable topsheet facing the top surface of the foam layer and a liquid impermeable backsheet facing the nonwoven comprising superabsorbent polymers.

13. An absorbent article according to claim 12, **characterised in that** outside of the periphery of the absorbent core, the topsheet is not bonded to the absorbent core or bonded in regions of the indented compressed portions.

14. An absorbent article according to claim 13, **characterised in that** the topsheet is a PE film or a laminate comprising a nonwoven and a PE film.

15. A method of making an absorbent core according to any of claims 1 to 10, comprising the steps of:
a) providing a polymer mixture comprising one or more reactants and/or polymers, preferably polyurethane;
b) applying or generating in-situ one or more fluid substances (typically a gas or an aqueous solution) such to promote a cell growth;
c) providing a nonwoven fabric;
d) passing at least some of the polymer mixture through the nonwoven fabric;
e) curing the polymer mixture;
f) profiling the polymer mixture;
g) drying the cured polymer mixture after profiling;
h) providing a nonwoven comprising superabsorbent polymers;
i) joining the nonwoven comprising superabsorbent polymers to the polymer mixture; and
j) creating openings at least across the polymer mixture.
